# EUROPÄISCHE PATENTSCHRIFT

(11) **EP 0 520 330 B1**
(45) Veröffentlichungstag und Bekanntmachung des Hinweises auf die Patenterteilung: **14.08.1996**
(21) Anmeldenummer: 92110374.3
(22) Anmeldetag: 19.06.1992
(51) Int. Cl.: A61F 13/02

(54) **Verpackte Verbände und medizinische Klebematerialien und deren Herstellung**
Packaged dressings and medical adhesive materials, and their production
Pansements et matériaux adhésifs médicaux emballés, et leur fabrication

(30) Priorität: 27.06.1991 DE 4121189
(43) Veröffentlichungstag der Anmeldung: 30.12.1992
(73) Patentinhaber: Beiersdorf Aktiengesellschaft, D-20245 Hamburg (DE)
(72) Erfinder: Gleim, Harald, W-2106 Bendestorf (DE); Kaden, Gert, W-2083 Halstenbek (DE)

(56) Entgegenhaltungen:
- EP-A- 0 101 298
- EP-A- 0 320 814
- US-A- 2 897 961
- US-A- 4 182 449
- US-A- 4 337 284

## Beschreibung

Die Erfindung betrifft Pflaster, insbesondere Wundpflaster oder Verbandmaterialien oder Klebematerialien für medizinische Zwecke, die mit einem Haftkleber versehen sind und sich in einer Verpackung befinden, der sie vor der Anwendung entnommen werden.

Verpackte Pflaster dieser Art sind mit unterschiedlichem Aufbau bekannt. Sie weisen jedoch bei der Anwendung mitunter unbefriedigende Eigenschaften auf.

So können lose, in briefartige Hüllen oder Umschläge eingelegte, mit Abdeckstreifen versehene Selbstklebepflaster aus der geöffneten Verpackung herausfallen. Außerdem müssen sie nach der Entnahme aus der Verpackung beidhändig appliziert werden, indem die im Bereich des Wundkissens überlappenden Trennpapiere zu entfernen sind. Dabei kann das Wundkissen durch verschmutzte und verkeimte Finger infiziert werden und die Klebemasse des Pflasters durch Berührung mit Schmutz oder Sekreten ihre Klebeeigenschaft verlieren.

Aus dem US-Patent 2,897,961 ist auch ein Selbstklebepflaster bekannt, das ohne Abdeckstreifen der Selbstklebemasse in einer Hülle liegt. Beim Aufreißen der Hülle sind beide Hände erforderlich und das dabei gleichzeitig frei zwischen den beiden Hüllenhälften hervortretende Pflaster mit seiner nun ungeschützten Wundauflage kann also nicht sofort gegriffen werden. Erst wenn die Packung fast vollständig geöffnet ist und nur noch das Ende des Pflasterstreifens an einer Packungsbahn haftet, kann der Pflasteranfang erfaßt werden. Auch hier können Klebemassen und Wundauflage verschmutzt werden.

Schließlich sind aus der europäischen Offenlegungsschrift EP-A-101 298 gefaltete Pflaster bekannt, die auf der Oberseite und auf der Unterseite selbstklebende Teile aufweisen, die mit zwei Abdeckblättern versehen sind, die mit ihren Außenseiten an zwei Hälften der Verpackungshülle kleben. Beim Aufreißen der Hüllenhälften lösen sich die Abdeckblätter von dem Pflaster und das Wundpflaster liegt ungeschützt offen. Bei der Entnahme des Pflasters kann dann aber eine Verschmutzung des Pflasters nicht sicher vermieden werden oder beim unmittelbaren Anlegen des Pflasters unter Zuhilfenahme der Hüllenteile ist der genaue Sitz des Pflasters nicht immer sicher gewährleistet.

Aufgabe der Erfindung ist es daher, verpackte selbstklebende Pflaster und Materialien der vorstehend genannten Art zu schaffen, die solche Nachteile nicht besitzen. So sollen insbesondere die genannten Pflaster und Materialien während des Öffnens der Verpackung und in der geöffneten Verpackung gegen Verschmutzung geschützt sein. Das bedeutet, daß die Selbstklebemasse sowie beispielsweise eine Wundabdeckung nicht ungeschützt freiliegen, bis das Pflaster oder die genannten Materialien entnommen werden, und die Entnahme soll einfach und sicher mit nur einer Hand erfolgen können.

Diese Aufgabe wird gelöst durch verpackte Pflaster, insbesondere Wundpflaster mit einer Wundabdeckung oder Verbandmaterialien oder Klebematerialien für medizinische Zwecke mit jeweils einem ebenen Träger, wobei der Träger mit einer Haftkleberschicht und gegebenenfalls einer Wundabdeckung versehen ist, die mit einem Abdeckstreifen bedeckt und geschützt sind, wobei der Abdeckstreifen an dieser Haftkleberschicht haftet, und wobei diese Pflaster oder Materialien von zwei Verpackungsblättern, die eine Verpackung bilden, umhüllt und eingeschlossen sind, wobei ein Verpackungsblatt über der Außenseite des genannten Abdeckstreifens liegt und auf seiner Innenseite mit einer Klebmasse oder Siegelmasse versehen und mit der Außenseite des Abdeckstreifens verklebt oder versiegelt ist, wodurch der Abdeckstreifen fester an dem Verpackungsblatt als an der Haftkleberschicht haftet, und wobei die beiden Verpackungsblätter in einem über die Fläche des Abdeckstreifens hinausgehenden Bereich miteinander verklebt oder versiegelt sind und sich die beiden Verpackungsblätter voneinander trennen lassen, wobei das nicht mit der Klebmasse oder Siegelmasse versehene Verpackungsblatt gegebenenfalls auch vollständig entfernt werden kann, und die genannten Pflaster oder Materialien mit dem Abdeckstreifen auf dem anderen, mit der Klebmasse oder Siegelmasse versehenen Verpackungsblatt in der Weise haften, daß die Haftkleberschicht und gegebenenfalls die Wundabdeckung bedeckt und geschützt sind.

Die so über die Haftkleberschicht mit dem verklebten oder versiegelten Abdeckstreifen fixierten erfindungsgemäßen Pflaster und Materialien können nun mit einer Hand gezielt abgenommen und appliziert werden, da sie sich mit der anderen Hand auf ihrer Unterlage, dem Verpackungsblatt, sicher halten lassen.

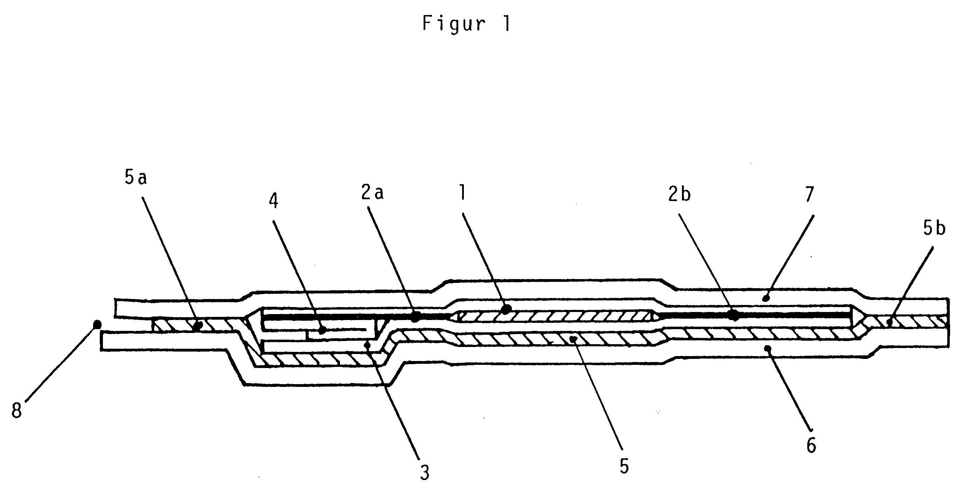

In einer bevorzugten Ausführungsform ist die Haftkleberschicht an der Stelle oder an dem Rand oder an dem Ende der Pflaster oder Materialien, an dem diese zuerst abgehoben werden, mit einer Anfaßhilfe, z.B. einer Schutzfolie versehen, die den Rand klebfrei macht, so daß der Träger mit der Anfaßhilfe, beispielsweise mit Daumen und Zeigefinger leichter erfaßt und abgehoben werden kann. Die Anfaßhilfe besteht vorzugsweise aus einem V-förmig gewinkelten und zurückgefalteten Kunststoffblatt oder Papierblatt, das mit einem Schenkel auf der Haftklebermasse haftet, während der andere Schenkel freiliegend zum Pflasterrand zurückgefaltet und vorzugsweise kürzer ist, d.h. unsymmetrisch V-förmig gewinkelt ist, so daß auch die Anfaßhilfe mühelos entfernt werden kann, wenn das erfindungsgemäße Pflaster oder Material appliziert ist. Eine bevorzugte Anfaßhilfe ist in Figur 1 dargestellt.

Vorzugsweise bedeckt diese Anfaßhilfe vom äußeren Rand her die Haftkleberschicht nur zu einem Teil und nicht vollflächig. Bevorzugt bedeckt sie etwa ein Viertel bis drei Viertel, insbesondere etwa die Hälfte bis etwa zwei Drittel der Fläche eines selbstklebenden Pflasterendes, insbesondere eines Wundpflasterendes oder des Endes des erfindungsgemäßen Materials, so daß z.B. neben einer Wundabdeckung die entsprechende, genügende Haftfläche verbleibt, die den sicheren Halt dieses Pflasterendes und damit des gesamten Pflasters auf der Abdeckstreifenunterlage gewährleistet.

Bei einer weiteren bevorzugten Ausführungsform der Erfindung ist die erfindungsgemäße Verpackung mit einer Öffnungshilfe versehen, die das Trennen der beiden Verpackungsblätter erleichtert. Diese Öffnungshilfe besteht aus zwei nicht miteinander verklebten oder versiegelten Rändern der Verpackungsblätter, die vorzugsweise nicht deckungsgleich übereinander liegen sondern leicht gegeneinander versetzt sind, so daß sie Anfaßlaschen bilden, die sich jeweils leicht erfassen und dann zum anschließenden Öffnen der Verpackung auseinander ziehen lassen.

Vorteilhafterweise sind die Öffnungshilfe der Verpackung und die Anfaßhilfe des Pflasters oder verpackten Materials am gleichen Ende, benachbart oder direkt nebeneinander angeordnet, so daß sie sich mühelos nacheinander erfassen lassen, wobei eine Hand an einer Lasche der Öffnungshilfe des die Klebmasse oder Siegelmasse tragenden Verpackungsblattes, das auch den Abdeckstreifen mit dem Pflaster trägt, verbleibt und die andere Hand nach Abzug des zweiten Verpackungsblattes sofort z.B. die Anfaßhilfe des verpackten Gegenstandes ergreifen kann.

Besonders bevorzugte Pflaster sind Wundpflaster, die in bekannter Weise einen biegsamen Träger, der mit einer Haftkleberschicht, d.h. mit einer druckempfindlichen Klebemasse versehen ist, besitzen und die ein Wundkissen tragen, das sich auf der mit dem Haftkleber beschichteten Seite befindet und dazu dient, Wundflüssigkeiten aufzunehmen.

Es können aber auch Pflaster in der erfindungsgemäßen Weise verpackt sein, die statt des Wundkissens andere Anordnungen tragen. Es können Arzneimittelträger sein, beispielsweise bei transdermalen Pflastern oder spezielle Anordnungen sein, wie bei keratolytischen Pflastern, Rheumapflastern oder Testpflastern. Es können ferner Verbandmaterialien, die zum Verschließen von Wunden dienen und auch Klebematerialien oder Klebestreifen für medizinische Zwecke, beispielsweise zum Fixieren von medizinischen Werkzeugen und Geräten Gegenstand der Erfindung sein.

Typische weitere erfindungsgemäße als Einzelpflaster verpackte Pflasterarten sind Augenpflaster, Fingerpflaster und Injektionspflaster.

Als Haftklebemasse der erfindungemäßen Haftkleberschicht können die für Pflaster und medizinische Zwecke üblichen Zusammensetzungen z.B. auf Kautschuk- oder Acrylat-Basis eingesetzt werden.

Die Abdeckstreifen dieser Selbstklebemassen sind beispielsweise in üblicher Weise vorzugsweise einseitig klebstoffabweisende oder klebstoffabweisend ausgerüstete Folien oder Papiere, die vorzugsweise die gesamte Fläche der Pflaster oder erfindungsgemäßen Materialien abdecken, und auf denen die Haftklebemassen ausreichend fest haften, auch wenn die obere Verpackungshülle entfernt ist. Ihre Rückseite ist vorzugsweise rauh und damit verklebbar oder siegelbar mit der erfindungsgemäßen Klebmasse oder Siegelmasse oder Siegelschicht und kann damit ausreichend fest mit der Innenseite der Verpackungshülle verbunden werden. So ist gewährleistet, daß bei einer Belastung wie dem Abziehen des Pflasters, sich das schwächer anhaftende Pflaster löst und der Abdeckstreifen mit der Verpackungshülle verbunden bleibt.

Besonders geeignet sind einseitig klebstoffabweisende, insbesondere silikonisierte Trennpapiere oder Trennfolien, wie sie üblicherweise für die Abdeckung von Haftklebern verwendet werden und deren unbehandelte Rückseite rauh oder verklebbar oder siegelbar ist. Solche Trennpapiere und Folien sind bekannt.

Als erfindungsgemäße Verpackungsblätter können übliche Verpackungsfolien und Papiere verwendet werden, die verklebbar oder siegelbar, insbesondere heißsiegelbar sind. Vorzugsweise ist ein Blatt durchsichtig oder transparent. Besonders geeignet sind Papier, Polypropylenfolie oder Polyethylentherephthalatfolie.

Ein bevorzugtes Papier ist ein einseitig glattes Kraftpapier, vorzugsweise mit einem Gewicht von 30 bis 100 g/m², insbesondere 35 bis 80 g/m², besonders bevorzugt aber 40 bis 60 g/m². Es kann auf der rauhen, nicht geglätteten Seite, aber auch auf der glatten Seite mit der Klebmasse oder Siegelmasse versehen sein, vorzugsweise aber auf der rauhen Seite.

Eine besonders geeignete Polypropylenfolie ist eine orientierte Polypropylenfolie, insbesondere eine biaxial gereckte Polypropylenfolie, vorzugsweise mit etwa gleichen Reckverhältnissen in beiden Richtungen.

Die Dicke der Folie kann beispielsweise 15 bis 50 µm, vorzugsweise 20 bis 40 µm und insbesondere 23 bis 30 µm betragen.

Werden Folien, insbesondere erfindungsgemäße Polypropylenfolien mit der Klebmasse oder Siegelmasse, insbesondere einer Heißsiegelmasse versehen, ist es günstiger, dickere Folien, etwa ab 30 µm Dicke einzusetzen.

Zur Verbesserung der Klebeigenschaften und der Siegelfähigkeit kann die Folie in üblicher Weise coronavorbehandelt oder mit einem dünnen Film eines siegelfähigen Polymers coextrudiert oder laminiert sein.

Als erfindungsgemäß mit der Klebemasse oder Siegelmasse versehenes Verpackungsblatt wird Papier bevorzugt. Es ist aber auch möglich, die Polypropylenfolie hierfür zu verwenden.

In einer bevorzugten Kombination ist ein Verpackungsblatt aus Papier und das andere aus Polypropylenfolie, wobei sich die Klebmasse oder die Siegelmasse auf dem Papier oder der Folie befinden kann.

Als besonders bevorzugte Kombination, insbesondere für Wundpflaster, die einzeln verpackt sind, wird ein mit Klebmasse oder Siegelmasse versehenes Papier zusammen mit einer Polypropylenfolie als zweitem Verpackungsblatt verwendet.

Eine weitere, im Hinblick auf die Gassterilisation günstige Kombination besteht aus zwei Papierblättern gemäß der Erfindung.

Als erfindungsgemäße Klebmassen für die Verbindung der Verpackungsbahnen und des Abdeckstreifens damit, können übliche Klebmassen dienen, die für die Verklebung der aufgeführten Materialien geeignet sind. Bevorzugt werden druckempfindliche Klebmassen, die mit unterschiedlicher Zusammensetzung in großer Zahl bekannt sind.

Als Siegelmassen können kaltsiegelnde oder kaltklebende Siegelmassen, die eine Verbindung durch Druckanwendung bewirken, oder Heißsiegelmassen, die eine Verbindung durch Wärme und Druck bewirken, verwendet werden.

Eine kaltsiegelnde Masse auf der Basis von Ethylen-Vinylacetat-Copolymeren, kaltreaktiven Harzen, Polyethylenderivaten wie Polyisobutylen und Füllstoffen wird bevorzugt.

Besonders bevorzugte Siegelmassen sind Heißsiegelmassen, und insbesondere geeignet sind Heißsiegelmassen auf der Basis von Ethylen-Vinylacetat-Copolymeren (EVA). Sie enthalten vorzugsweise Polyethylen, insbesondere niedermolekulares Polyethylen und/oder Füllstoffe wie z.B. Titandioxid.

Diese Siegelmassen haben die besondere Eigenschaft, gegen das unbeschichtete Verpackungsblatt ablösbar oder abschälbar (peelable) zu siegeln und gleichzeitig den das Wundkissen und die klebenden Pflasterteile schützenden Abdeckstreifen festzusiegeln.

Vorteilhafterweise wird die Klebmasse oder die Siegelmasse auf ein Verpackungsblatt oder bei der Massenproduktion auf eine Verpackungsbahn aufgetragen. Dabei ist es möglich, einen Randstreifen unbeschichtet zu lassen, der an der späteren Verpackung als Öffnungshilfe dient oder zusammen mit dem zweiten Verpackungsblatt die Öffnungshilfe bildet, indem auch an diesem Blatt ein freier Griffrand verbleibt. Beschichtete Ränder können aber auch gefaltet und/oder aufeinander geklebt oder gesiegelt sein oder mit zusätzlichen Streifen abgedeckt sein.

Zweckmäßigerweise ist die gesamte gewünschte Fläche des Verpackungsblattes mit der Klebmasse oder der Siegelmasse versehen. Dabei kann eine kontinuierliche Schicht gebildet werden, aber auch ein rasterartiger, partieller Auftrag erfolgen, demzufolge die Masse separate Punkte bildet. Die kontinuierliche Schicht kann auch Unterbrechungen und freie Flächen enthalten und z.B. eine Gitterstruktur bilden. Die Auftragsmenge beträgt vorzugsweise 4 bis 30 g/m², insbesondere 6 bis 20 g/m², besonders bevorzugt aber 7 bis 11 g/m².

In Figur 1 ist beispielsweise ein bevorzugtes verpacktes Wundpflaster im Längsschnitt mit vergrößerten Dicken der Schichten dargestellt. Es besitzt einen Träger mit einem Wundkissen darauf 1, sowie Haftkleberschichten 2a, 2b an beiden Trägerseiten, die auf dem Abdeckstreifen 3 und der unsymmetrisch V-förmig gewinkelten, zurückgefalteten Anfaßhilfe 4 haften. Der Abdeckstreifen 3 ist mit der Klebmasseschicht oder Siegelmasseschicht 5 auf das Verpackungsblatt 6 geklebt oder gesiegelt. An den Rändern 5a, 5b, die über das eingelegte, abgedeckte Pflaster (1 - 4) hinausragen, ist das unbeschichtete zweite Verpackungsblatt 7 aufgeklebt oder aufgesiegelt. An einem Rand mit der Öffnungshilfe 8 ist keine Klebmasse oder Siegelmasse aufgetragen und das das Pflaster tragende Verpackungsblatt 6 ragt über das Verpackungsblatt 7 hinaus. Andere erfindungsgemäße Materialien sind analog verpackt.

Die Herstellung der erfindungsgemäßen verpackten Pflaster und Materialien kann auf übliche Anlagen erfolgen und geschieht vorzugsweise folgendermaßen:

In einem ersten, separaten Schritt werden die zu verpackenden Pflaster und Materialien, ausgerüstet mit dem Abdeckstreifen, in bekannter Weise hergestellt. Die Pflasterkanten werden dabei vorzugsweise zusammen mit dem Abdeckstreifen ausgestanzt.

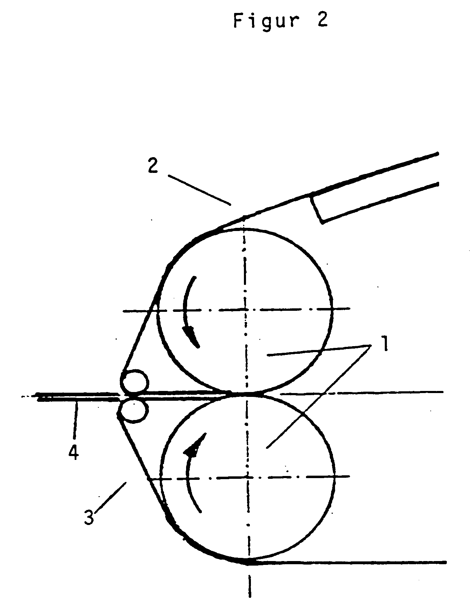

In einem zweiten Schritt werden diese zu verpackenden Gegenstände in eine Klebestation oder Siegelstation (beispielsweise wie in Figur 2 angegeben) gegeben, der eine erste mit Klebmasse oder mit Siegelmasse beschichtete und eine zweite, unbeschichtete Verpackungsbahn zugeführt werden, die die Verpackungsblätter gemäß der Erfindung ergeben, wobei die beschichtete Seite nach innen, zur zweiten Bahn gerichtet ist. Die zu verpackenden Gegenstände werden so zwischen diese Verpackungsbahn geführt, daß der Abdeckstreifen zur beschichteten Bahn gerichtet ist. Unter Anwendung von Druck und gegebenenfalls Wärme werden dann die Verpackungsbahnen und der Abdeckstreifen vorzugsweise in einem Arbeitsvorgang miteinander verklebt oder versiegelt. Die Beschichtung der ersten Bahn verbindet sich gleichzeitig, in einem Arbeitsvorgang, mit der Rückseite des Abdeckstreifens und vollflächig mit der unbeschichteten, über Pflaster und Materialien hinausragenden zweiten Verpackungsbahn.

Gegenstand der Erfindung ist auch ein Verfahren zur Herstellung erfindungsgemäßer verpackter Pflaster, Verbandmaterialien und Klebematerialien, das dadurch gekennzeichnet ist, daß Pflaster insbesondere Wundpflaster mit einer Wundabdeckung oder Verbandmaterialien oder Klebematerialien für medizinische Zwecke mit jeweils einem ebenen Träger, wobei der Träger mit einer Haftkleberschicht und gegebenenfalls einer Wundabdeckung versehen ist, die mit einem Abdeckstreifen bedeckt und geschützt sind, wobei der Abdeckstreifen an dieser Haftkleberschicht haftet, zwischen eine mit Klebmasse oder mit Siegelmasse beschichtete und eine unbeschichtete Verpackungsbahn geführt werden, wobei die beschichtete Seite innen, zwischen den Bahnen liegt und der Abdeckstreifen der Pflaster und Materialien zur beschichteten Bahn gerichtet ist, und der Abdeckstreifen und die Verpackungsbahnen unter Anwendung von Druck und gegebenenfalls von Wärme vorzugsweise in einem Arbeitsvorgang miteinander verklebt oder versiegelt werden.

Vorzugsweise werden in einem zweiten Schritt diese zu verpackenden Gegenstände in eine Siegelstation gegeben, der eine mit Siegelmasse beschichtete erste und eine unbeschichtete zweite Verpackungsbahn zugeführt werden, die die Verpackungsblätter gemäß der Erfindung ergeben. Beim Einlaufen des Pflasters oder Materials in die Siegelstation wird beispielsweise von oben die erste beschichtete und gegebenenfalls auf Siegeltemperatur erwärmte Verpackungsbahn und von unten die zweite unbeschichtete Verpackungsbahn zugeführt. Die gegebenenfalls schmelzflüssige Beschichtung der ersten Bahn verbindet sich gleichzeitig, in einem Arbeitsgang, mit der Rückseite des Abdeckstreifens und vollflächig mit der unbeschichteten, über Pflaster und Materialien hinausragenden zweiten Verpackungsbahn.

Vorzugsweise werden Pflaster, Wundpflaster oder andere schmale Materialien quer zur Laufrichtung der Verpackungsbahnen eingegeben, so daß siegelfreie Bahnränder die spätere Öffnungshilfe bilden können. Dies läßt sich aber auch durch Umfalten oder Abdecken der Ränder mit zusätzlichen Streifen erreichen. Außerdem kann die Herstellung schneller erfolgen.

Anschließend können Einzelpackungen durch Vorperforation oder Schnitt durch die verklebten oder versiegelten Verpackungsbahnen hindurch erhalten werden. Weiterhin kann sich die Sterilisation, beispielsweise mit energiereicher Strahlung anschließen. Geeignet ist die Bestrahlung mit Gammastrahlen mit z.B. 2,5 Mrad absorbierter Dosis oder die Behandlung mit sterilisierenden Gasen wie Ethylenoxid.

In Figur 2 ist beispielsweise eine Siegelstation 1 mit beheizter, oberer Bahn 2, deren äußere Oberseite beschichtet ist und unterer Bahn 3 dargestellt, in die ein erfindungsgemäßes, abgedecktes Pflaster oder erfindungsgemäße Materialien 4 eingegeben werden.

Für die bevorzugte Heißsiegelung kann eine beheizte, obere Bahn in einer Vorwärmstrecke, vorzugsweise bei einer Temperatur von 110° bis 180°C, insbesondere bei 120 bis 160°C, vorgeheizt und dann in der eigentlichen Siegelstation mit einer beheizten Stahlwalze bei niedrigerer Temperatur, vorzugsweise bei 65 bis 100°C, insbesondere 70° bis 90°C, und unbeheizter Gummigegenwalze mit dem Abdeckstreifen und der weiteren, unbeschichteten Bahn gesiegelt werden, so daß die Pflaster oder andere erfindungsgemäße Materialien festgesiegelt und dicht eingeschlossen sind.

Die erfindungsgemäßen verpackten Pflaster, Wundpflaster, Verbandmaterialien und Klebematerialien für medizinische Zwecke sind in überraschender Weise während des Öffnens der Verpackung und auch noch in der geöffneten Verpackung gegen Verschmutzung geschützt und lassen sich außerdem noch einfach mit einer Hand ergreifen und applizieren. Sie sind, da sie eben auf einer Verpackungshälfte aufliegen, mit großen Teilen ihrer Fläche festhaftend fixiert, wobei auch ein Wundkissen oder dergleichen abgedeckt bleibt. Damit wird es auch möglich, mehrere gemeinsam verpackte Gegenstände wie z.B. Pflaster, die nacheinander verwendet werden sollen, durch einen einzigen Öffnungsvorgang freizulegen und eine kurze Zeit bereitzuhalten, da nicht die Gefahr der Verschmutzung besteht.

Auch das Herstellungverfahren ist überraschend einfach und es gestattet sehr hohe Stückzahlen, so daß die hier erforderliche Massenproduktion möglich ist.

Die folgenden Beispiele dienen zur Erläuterung der Herstellung der erfindungsgemäßen Pflaster und Materialien:

### Beispiel 1

Ein Wundpflaster, das aus einem biegsamen Träger, der mit einer Haftkleberschicht, einem Wundkissen und einem silikoniserten Abdeckstreifen aus Papier mit rauher Außenseite besteht und eine V-förmig gewinkelte asymmetrische Anfaßhilfe an einem Pflasterende trägt, wie in Figur 1 dargestellt, wird in bekannter Weise hergestellt. Zusammen mit dem Abdeckstreifen werden die Pflasterkanten ausgestanzt.

Das in dieser Weise vorgefertigte, abgedeckte Pflaster wird an eine Siegelstation entsprechend Figur 2 übergeben, wobei der Abdeckstreifen auf der Oberseite liegt und damit zur Beschichtung der beschichteten Bahn gerichtet ist. In der Siegelstation, bestehend aus einer Vorwärmstrecke mit einer Temperatur von 120°C und der eigentlichen Siegelstation mit beheizter Stahlwalze mit einer Temperatur von 80°C und unbeheizter Gummiwalze werden die Pflaster kontinuierlich mit einer Stückzahl von 300 Stück/min festgesiegelt und eingesiegelt. Dazu wird von oben eine einseitig geglättete Papierbahn (45 g/m²) über die Vorwärmstrecke zugeführt, die auf der nicht geglätteten Seite mit einer Heißsiegelmasse auf der Basis von EVA und niedermolekularem Polyethylen mit einer Auftragsmenge von 9 g/m² beschichtet ist. Von unten wird eine biaxial gereckte Polypropylen- Verpackungsfolie (Typ OPP) mit einer Dicke von 20 µm zugeführt.

Nach Verlassen der Siegelstation wird das Pflaster in der Schneidestation durch Vorperforation und/oder Schnitt einzeln, als Doppelstrip oder als Viererstrip konfektioniert oder als Rollenware verarbeitet.

### Beispiel 2

Es wird wie in Beispiel 1 angegeben verfahren aber mit dem Unterschied, daß die Temperatur der Vorwärmstrecke 150°C beträgt und 600 Pflaster pro Minute eingesiegelt werden.

### Beispiel 3

Es wird wie in Beispiel 1 angegeben verfahren, aber mit dem Unterschied, daß von oben eine Polypropylenfolie (OPP) mit einer Dicke von 35 µm zugeführt wird, die mit 11 g/m² der Heißsiegelmasse beschichtet ist und von unten ein einseitig geglättetes Papier mit einem Gewicht von 40 g/m² so zugeführt wird, daß die geglättete Seite die Pflasteraußenseite bildet.

### Beispiel 4

Es wird wie in Beispiel 1 angegeben verfahren, aber mit dem Unterschied, daß von oben eine einseitig geglättete Papierbahn mit einem Gewicht von 45 g/m² und einer Beschichtung der Heißsiegelmasse von 6 g/m² auf der nicht geglätteten Seite zugeführt wird und von unten ein einseitig glattes Papier von 45 g/m².

## Patentansprüche

1. Verpackte Pflaster, insbesondere Wundpflaster mit einer Wundabdeckung oder Verbandmaterialien oder Klebematerialien für medizinische Zwecke mit jeweils einem ebenen Träger (1), wobei der Träger mit einer Haftkleberschicht (2a, 2b) und gegebenenfalls einer Wundabdeckung versehen ist, dadurch gekennzeichnet, daß diese mit einem Abdeckstreifen (3) bedeckt und geschützt sind, wobei der Abdeckstreifen (3) an dieser Haftkleberschicht (2a, 2b) haftet, und wobei diese Pflaster oder Materialien von zwei Verpackungsblättern (6, 7), die eine Verpackung bilden, umhüllt und eingeschlossen sind, wobei ein Verpackungsblatt über der Außenseite des genannten Abdeckstreifens liegt und auf seiner Innenseite mit einer Klebmasse oder Siegelmasse (5) versehen und mit der Außenseite des Abdeckstreifens verklebt oder versiegelt ist, wodurch der Abdeckstreifenn (3) fester an dem Verpackungsblatt als an der Haftkleberschicht haftet, und wobei die beiden Verpackungsblätter (6, 7) in einem über die Fläche des Abdeckstreifens (3) hinausgehenden Bereich miteinander verklebt oder versiegelt sind und sich die beiden Verpackungsblätter (6, 7) voneinander trennen lassen, wobei das nicht mit der Klebmasse oder Siegelmasse versehene Verpackungsblatt gegebenenfalls auch vollständig entfernt werden kann, und die genannten Pflaster oder Materialien mit dem Abdeckstreifen (3) auf dem anderen, mit der Klebmasse oder Siegelmasse (5) versehenen Verpackungsblatt in der Weise haften, daß die Haftkleberschicht und gegebenenfalls die Wundabdeckung bedeckt und geschützt sind.

2. Verpackte Pflaster und Materialien gemäß Anspruch 1, dadurch gekennzeichnet, daß sie eine Anfaßhilfe besitzen.

3. Verpackte Pflaster und Materialien gemäß Anspruch 1, dadurch gekennzeichnet, daß sie eine Anfaßhilfe besitzen, die nur einen Teil der Haftkleberschicht abdeckt.

4. Verpackte Pflaster und Materialien gemäß Anspruch 1, dadurch gekennzeichnet, daß der Abdeckstreifen ein einseitig silikonisiertes Trennpapier ist.

5. Verpackte Pflaster und Materialien gemäß Anspruch 1, dadurch gekennzeichnet, daß die Verpackungsblätter aus Papier oder Polypropylenfolie bestehen.

6. Verpackte Pflaster und Materialien gemäß Anspruch 1, dadurch gekennzeichnet, daß das mit Klebmasse oder Siegelmasse versehene Verpackungsblatt aus Papier und das andere Verpackungsblatt aus Polypropylen besteht.

7. Verpackte Pflaster und Materialien gemäß Anspruch 1, dadurch gekennzeichnet, daß die Siegelmasse eine Heißsiegelmasse auf der Basis eines Ethylen-Vinylacetat-Copolymers ist.

8. Verpacktes Wundpflaster gemäß Anspruch 1 mit einem Träger und einem Wundkissen darauf (1), sowie Haftkleberschichten (2a), (2b) an beiden Trägerseiten, die auf dem Abdeckstreifen (3) und der unsymmetrisch V-förmig gewinkelten, zurückgefalteten Anfaßhilfe (4) haften, wobei der Abdeckstreifen (3) mit der Klebmasseschicht oder Siegelmasseschicht (5) auf das Verpackungsblatt (6) geklebt oder gesiegelt ist, und an den Rändern (5a), (5b), die über das eingelegte, abgedeckte Pflaster hinausragen, das unbeschichtete zweite Verpackungsblatt (7) aufgeklebt oder aufgesiegelt ist, und an einem Rand mit der Öffnungshilfe (8) keine Kleb- oder Siegelmasse aufgetragen ist und das das Pflaster tragende Verpackungsblatt (6) über das Verpackungsblatt (7) hinausragt.

9. Verfahren zur Herstellung verpackter Pflaster, Verbandmaterialien und Klebematerialien gemäß Anspruch 1, dadurch gekennzeichnet, daß Pflaster, insbesondere Wundpflaster mit einer Wundabdeckung oder Verbandmaterialien oder Klebematerialien für medizinische Zwecke mit jeweils einem ebenen Träger, wobei der Träger mit einer Haftkleberschicht und gegebenenfalls eine Wundabdeckung versehen ist, die mit einem Abdeckstreifen bedeckt und geschützt sind, wobei der Abdeckstreifen an dieser Haftkleberschicht haftet, zwischen eine mit Klebmasse oder mit Siegelmasse beschichtete und eine unbeschichtete Verpackungsbahn geführt werden, wobei die beschichtete Seite innen, zwischen den Bahnen liegt und der Abdeckstreifen der Pflaster und Materialien zur beschichteten Bahn gerichtet ist, und der Abdeckstreifen und die Verpackungsbahnen unter Anwendung von Druck und gegebenenfalls von Wärme gegebenenfalls in einem Arbeitsvorgang miteinander verklebt oder versiegelt werden.

## Claims

1. Packaged plasters, especially adhesive plasters with a wound covering or dressing materials or adhesive materials for medical purposes, each having a flat support (1) provided with a layer (2a, 2b) of pressure-sensitive adhesive and, if desired, with a wound cover, characterized in that said layer and cover are covered and protected by a cover strip (3) which adheres to said layer (2a, 2b) of pressure-sensitive adhesive, these plasters or materials being enveloped and enclosed by two packaging leaves (6, 7) which form a pack, one packaging leaf lying over the outside of the abovementioned cover strip and being provided on its inside with an adhesive composition or sealing composition (5) and being bonded or sealed to the outside of the cover strip, so that the cover strip (3) adheres more firmly to the packaging leaf than to the layer of pressure-sensitive adhesive, and the two packaging leaves (6, 7) being bonded or sealed to one another in a region which extends beyond the area of the cover strip (3), and the two packaging leaves (6, 7) being separable from one another, it also being possible for the packaging leaf which is not provided with the adhesive composition or sealing composition to be removed completely, if desired, and the said plasters or materials bearing the cover strip (3) adhering to the other packaging leaf, provided with adhesive composition or sealing composition (5), in such a way that the layer of pressure-sensitive adhesive and, if desired, the wound covering are covered and protected.

2. Packaged plasters and materials according to Claim 1, characterized in that they possess a gripping aid.

3. Packaged plasters and materials according to Claim 1, characterized in that they possess a gripping aid which covers only part of the layer of pressure-sensitive adhesive.

4. Packaged plasters and materials according to Claim 1, characterized in that the cover strip is a release paper which is silicone-treated on one side.

5. Packaged plasters and materials according to Claim 1, characterized in that the packaging leaves consist of paper or polypropylene film.

6. Packaged plasters and materials according to Claim 1, characterized in that the packaging leaf which is provided with adhesive or sealing composition consists of paper and the other packaging leaf consists of polypropylene.

7. Packaged plasters and materials according to Claim 1, characterized in that the sealing composition is a heat-sealing composition based on an ethylene-vinyl acetate copolymer.

8. Packaged adhesive plaster according to Claim 1, with a support and a compress thereon (1), and with layers (2a), (2b) of pressure-sensitive adhesive on both sides of the support, which adhere to the cover strip (3) and to the folded-back gripping aid (4) which is angled in the form of an asymmetric V, the cover strip (3) bearing the layer (5) of sealing composition or layer of adhesive composition being bonded or sealed to the packaging leaf (6), and the uncoated second packaging leaf (7) being applied by bonding or sealing to the edges (5a), (5b) which extend beyond the inserted, covered plaster, and no adhesive or sealing composition being applied on one edge with the opening aid (8), and the packaging leaf (6) which bears the plaster extending beyond the packaging leaf (7).

9. Process for the production of packaged plasters, dressing materials and adhesive materials according to Claim 1, characterized in that plasters, especially adhesive plasters having a wound cover or dressing materials or adhesive materials for medical purposes, each having a flat support provided with a layer of pressure-sensitive adhesive and, if desired, with a wound cover, said layer and cover being covered and protected by a cover strip which adheres to said layer of pressure-sensitive adhesive, are guided between a packaging web, which is coated with adhesive composition or with sealing composition, and an uncoated packaging web, the coated side lying on the inside, between the webs, and the cover strip of the plasters and materials facing the coated web, and the cover strip and the packaging webs are bonded or sealed to one another with the application of pressure and, if desired, of heat, if appropriate in one operation.

## Revendications

1. Emplâtres emballés, notamment des emplâtres pour lésions avec un recouvrement de plaies ou des matériaux de pansement ou des matériaux adhésifs destinés á un usage médical ayant à chaque fois un support plat (1), le support étant muni d'une couche auto-adhésive (2a, 2b) et le cas échéant d'un recouvrement de plaies caractérisés par le fait que ceux-ci sont recouverts et protégés par une bande de protection (3), la bande de protection (3) adhérant sur cette couche autoachésive (2a, 2b), et ces emplatres ou matériaux étant entourés de et enfermés dans deux feuilles d'emballage (6, 7) qui forment un emballage, une feuille d'emballage reposant sur le côté extérieur de ladite bande de protection et étant munie d'une matiére adhésive ou d'une matiére de scellement (5) sur son côte intérieur et étant collée ou scellée au coté extérieur de la bande de protection, la bande de protection (3) adhérant ainsi plus fortement á la feuille d'emballage qu'á la couche auto-adhésive, et les deux feuilles d'emballage (6, 7) étant collées ou scellées ensemble dans une zone dépassant la surface de la bande de protection (3) et les deux feuilles d'emballage (6, 7) se laissant séparer l'une de l'autre, la feuille d'emballage non munie de la matiére adhésive ou de la matiére de scellement pouvant également étre complètement retirée si nécessaire, et lesdits emplâtres ou matériaux munis de la bande de protection (3) adhèrent á l'autre feuille d'emballage munie de la matière adhésive ou de la matiére de scellement (5) de telle facon que la couche auto-adhésive et, le cas échéant, le recouvrement de plaies, soient recouverts et protégés.

2. Emplâtres et matériaux emballés conformes á la revendication 1, caractérisés par le fait qu'ils possédent un dispositif permettant de les prendre en main.

3. Emplâtres et matériaux emballés conformes á la revendication 1, caractérisés par le fait qu'ils possédent un dispositif permettant de les prendre en main, lequel ne recouvre au'une partie de la couche autoadhésive.

4. Emplâtres et matériaux emballés conformes á la revendication 1, caractérisés par le fait que la bande de protrection est, d'un coté, un papier de séparation siliconé.

5. Emplâtres et matériaux emballés conformes á la revendication 1, caractérisés par le fait que les feuilles d'emballage sont composées de papier ou de film de polypropyléne.

6. Emplâtres et matériaux emballés conformes á la revendication 1, caractérisés par le fait que la feuille d'emballage munie de matiére adhésive ou de matiére de scellement est composée de papier et que l'autre feuille d'emballage est composée de polypropyléne.

7. Emplâtres et matériaux emballés conformes á la revendication 1, caractérisés par le fait que la matiére de scellement est une matiére de thermoscellement á base d'un copolymére éthylène - acétate de vinyle.

8. Emplâtres pour lésions emballés conforme á la revendication 1 muni d'un support et d'un recouvement de plaies sur celui-ci (1), ainsi que de couches auto-adhésives (2a), (2b) des déux cotés du support, lesquelles adhérent sur la bande de protection (3) et sur le dispositif de prise en main (4) non symétrique enroulé en forme de V et replié, la bande de protection (3) étant collée ou scellée à la couche de matiére adhésive ou la couche de matiére de scellement (5) sur la feuille d'emballage (6), et la deuciéme feuille d'emballage (7) non enduite étant collée ou scellée sur les bords (5a), (5b) qui dépassent du emplatre recouvert qui se trouve á l'intérieur, et aucune matiére de collage ou de scellement n' étant appliquée sur un coté muni de l'accessoire d'ouverture (8) et la feuille d'emballage (6) qui porte le emplatre dépassant de la feuille d'emballage (7).

9. Procédé de fabrication de emplatres, de matériaux de pansement et de matériaux adhésifs emballés conformes á la revendication 1, caractérisé par le fait que les emplatres, notamment les emplatres pour lésions avec un revouvrement de plaies ou des matériaux de pansement ou des matériaux adhésifs destinés á un usage médical sont á chaque fois munis d'un support plat, le support étant muni d'une couche auto-adhésive et, le cas échéant d'un recouvrement de plaies lesquels sont recouverts et protégés par une bande de protection, la bande de protection adhérant sur cette couche auto-adhésive, sont guidés entre une feuille d'emballage enduite d'une matiére adhésive ou d'une matiére de scellement et une feuille d'emballage non enduite, le coté enduit se trouvant á l'intérieur entre les bandes, et la bande de protection des emplatres et des matériaux étant orientée vers la feuille enduite, et la bande de protection et les feuilles d'emballage étant collées ou scellées ensemble en utilisant la pression et, le cas échéant, de la chaleur, éventuellement au cours d'une phase de traváil.
